# EUROPÄISCHE PATENTSCHRIFT

(11) **EP 1 549 258 B1**
(45) Veröffentlichungstag und Bekanntmachung des Hinweises auf die Patenterteilung: **20.04.2011**
(21) Anmeldenummer: 03750508.8
(22) Anmeldetag: 10.09.2003
(51) Int. Cl.: A61F 2/40

(54) **BAUSATZ FÜR DIE THERAPIE VON KAPITALEN UND SUBKAPITALEN HUMERUSFRAKTUREN**
KIT FOR THE THERAPY OF CAPITAL AND SUBCAPITAL HUMERUS FRACTURES
TROUSSE POUR LE TRAITEMENT DE FRACTURES CAPITALES ET SOUS-CAPITALES DE L'HUMERUS

(30) Priorität: 10.09.2002 DE 20214654 U; 12.11.2002 DE 20217725 U
(43) Veröffentlichungstag der Anmeldung: 06.07.2005
(73) Patentinhaber: Tantum AG, 24537 Neumünster (DE)
(72) Erfinder: NIEMAX, Heinrich, 24536 Neumünster (DE); JENSEN, Harm-Iven, 24214 Noer (DE)
(74) Vertreter: Wenzel & Kalkoff
(86) Internationale Anmeldenummer: PCT/EP2003/010052
(87) Internationale Veröffentlichungsnummer: WO 2004/024029

(56) Entgegenhaltungen:
- EP-A- 0 940 126
- FR-A- 2 758 256
- US-A- 5 776 194
- US-B1- 6 171 341
- US-B1- 6 193 758

## Beschreibung

Die Erfindung betrifft einen Bausatz für die Therapie von kapitalen und subkapitalen Humerusfrakturen, umfassend einen von proximal in die Markhöhle des Humerus einführbaren, mit seiner Längsachse sich lang erstreckenden intramedullären Nagelschaft, wenigstens eine an dem proximalen Ende des Nagelschafts angeordnete, als Prothesengelenkkopf ausgebildete Kopfkalotte und wenigstens ein Verbindungsmittel, das die Kopfkalotte mit dem Schaft verbindet.

Aus US 5 776 194 sind Humeruskopfprothesen mit besonderen austauschbaren Prothesenkopf-Ansatzstücken bekannt. Lage und Ausrichtung jedes Prothesenkopfes sind mit einem Ansatzstück vorgegeben und nicht variierbar. Das Ansatzstück bildet den austauschbaren proximalen Teil eines Schaftes, dessen unterer, in einen intramedullären Humeruskanal einzuführender Teil nur mit Verriegelungsmitteln verwendbar ist. Zur Nutzung des oberen Schaftteils für einen Nagel zum Versorgen von kapitalen oder subkapitalen Humerusfrakturen ist anstelle eines Prothesenkopf-Ansatzstückes ein besonderes Ansatzstück zur Verwendung mit Versorgungsschrauben anzubringen. Andere bekannte Humeruskopfprothesen (z.B. DE-A1-195 48 154, DE-A1-196 30 298, DE-A1-198 41 612, DE 195 09 037) umfassen einen in den Humeruskanal des Oberarms einführbaren Schaft oder Stiel sowie daran festsetzbare, gegebenenfalls austauschbare kugelabschnittsförmige Gelenkköpfe. Die Befestigung der Gelenkköpfe erfolgt mittels Steckverbindung an einer Kopfplatte, einem Kragen, einer Stirnfläche, einem Kopfhalsteil oder einer Zwischenscheibe. Insbesondere ist ein Schaft mit einem den Gelenkkopf tragenden Schiebeeinsatz bekannt (DE-A1-195 48 154), und es ist vorgeschlagen worden, ein Kopfhalsteil als Winkeladapter vorzusehen (DE 195 09 037), wobei der Schaft zum Arretieren des Adapters besonderer Anpassung bedarf. Auch ist es bekannt, Schulterendoprothesen modular zusammenzusetzen. Zum Beispiel umfaßt ein bekannter Bausatz (EP-B1-0613 353) mehrere Schäfte sowie zum Ersatz des Humeruskopfes vorgesehene Kopfteile. Weiterhin sind Humerusnägel bekannt (z. B. EP 0 565 812). Ein solcher Nagel umfaßt ein von proximal in die Markhöhle des Humerus einführbaren Nagelschaft und von lateral durch Querdurchbohrungen hindurchführbare Knochenschrauben zum Versorgen von kapitalen und subkapitalen Humerusfrakturen.

Ziele der Erfindung bestehen darin, ein universelles modulares System zur Therapie von kapitalen und subkapitalen Humerusfrakturen zu schaffen. Dabei soll wenigstens ein einteiliges Basisteil des Systembausatzes sowohl mit Knochenschrauben zur Versorgung von Frakturen als auch als Teil einer Humeruskopfprothese verwendbar sein. Eine modulare Humeruskopfprothese, wie sie mit dem erfindungsgemäßen Bausatz herstellbar ist, soll einfach gestaltet sein und einfache Operations-Technik gewährleisten.

Um ein universelles modulares System zu erhalten, sieht die Erfindung einen Bausatz für die Therapie von kapitalen und subkapitalen Humerusfrakturen nach Anspruch 1 vor. Der Bausatz umfasst folgende zueinandergehörige Implantat-Einzelteile: Wenigstens einen von proximal in die Markhöhle des Humerus einführbaren, mit seiner Längsachse sich lang erstreckenden Nagelschaft einer intramedullären Osteosyntheseeinrichtung, wobei der Nagelschaft wenigstens eine Querdurchbohrung für wenigstens eine zugehörige Versorgungsschraube zum Versorgen kapitaler und/oder subkapitaler Humerusfrakturen aufweist; wenigstens eine an dem proximalen Ende des Nagelschafts befestigbare, mittels Ausformung gegen den Nagelschaft setzbare, als Humerus-Prothesengelenkkopf ausgebildete Kopfkalotte; wenigstens ein Verbindungsmittel zum Verbinden jeder Kopfkalotte mit dem Nagelschaft und wenigstens eine Versorgungsschraube und wenigstens eine Verriegelungsschraube, wobei der genannte Nagelschaft, der mit wenigstens einer Querdurchbohrung in Verbindung mit zugehöriger Versorgungsschraube zum Versorgen kapitaler und/oder subkapitaler Humerusfrakturen ausgebildet ist, an seinem proximalen Ende im Bereich wenigstens einer Versorgungs-Querdurchbohrung zugleich zur Verbindung mit der wenigstens einen gegen den Nagelschaft setzbaren Kopfkalotte des Bausatzes ausgebildet ist.

Der Nagelschaft ist ungeteilter Bestandteil einer intramedullären Marknagel-Osteosyntheseeinrichtung ist, wobei der Nagelschaft mit wenigstens einer Querdurchbohrung für eine zugehörige Versorgungsschraube zum Versorgen kapitaler und/oder subkapitaler Humerusfrakturen ausgebildet ist. Auch die Humeruskopfprothese ist Bestandteil des erfindungsgemäßen Bausatzes.

Erfindungsgemäß werden Bestandteile einer Osteosyntheseeinrichtung genutzt, um wahlweise kapitale oder subkapitale Humerusfrakturen zu versorgen und/oder Teile des Oberarmknochens, nämlich Kopf sowie gegebenenfalls Hals und benachbarte Teile mit einem Dauerimplantat zu ersetzen. Basis der erfindungsgemäßen Gegenstände ist der von proximal in die Markhöhle des Humerus einführbare ungeteilte Marknagelschaft der Osteosyntheseeinrichtung. Erfindungsgemäß wird erreicht, daß der Nagelschaft wahlweise in Verbindung mit zugehörigen Schrauben die Osteosyntheseeinrichtung und/oder mit der Kopfkalotte die Humeruskopfprothese bildet. Sämtliche Teile lassen sich in dem erfindungsgemäßen Bausatz steril bereit halten. Dieser baut kompakt und mit optimal reduzierter Zahl seiner Einzelteile. Durch geeignete Wahl von Knochenund Versorgungsschrauben läßt sich eine individuelle Anpassung an versorgbare Frakturen vornehmen. Mit der oder den zu ein und demselben einteiligen Nagelschaft zugehörigen Knochenschrauben lassen sich heilbare Frakturen auch mit größerer Zahl von Frakturstücken versorgen. Das mehrteilige modulare Bausystem erlaubt darüber hinaus - auch während der Operation - die Wahl geeigneter Therapie in Anpassung an Art und Ausmaß der Fraktur dadurch, daß der Gelenkkopf ersetzt werden kann. Häufig kann die operative Therapie überhaupt erst während der Operation bestimmt werden. Wenn zum Beispiel Trümmerfrakturen des gelenkbildenden Humeruskopfes besonders starke Zersplitterung aufweisen, oder wenn es sich erweist, daß Nerven und Blutversorgung unterbrochen sind, ist eine künstliche Kopfkalotte zu implantieren. Der Nagelschaft der erfindungsgemäßen Einrichtungen läßt sich besonders einfach von proximal in die Markhöhle des Humerus einbringen.

In bevorzugter erfindungsgemäßer Gestaltung wird an der Unterseite der genannten Kopfkalotte wenigstens eine eine Führung bildende Ausformung ausgebildet, mittels der die Kopfkalotte im Aufsitz gegen den Nagelschaft setzbar ist, und ein lateral an dem Nagelschaft angreifendes Verbindungsmittel wird vorgesehen, das in gelöstem Zustand Kopfkalotte und Nagelschaft lose und unverlierbar mit Justier-Bewegungsspiel für die Kopkalotte zum Ausrichten derselben miteinander verbindet, und das in gespanntem Zustand die Verbindung zwischen Kopfkalotte und Nagelschaft fixiert. Vorzugsweise kann das lateral an dem Nagelschaft angreifende, zum Ausrichten der Kopfkalotte vorgesehene Verbindungsmittel ein einziges stabförmiges Verbindungselement zum losen und fixierenden Verbinden der Kopfkalotte mit dem Nagelschaft umfassen. Eine bevorzugte Gestaltung besteht darin, daß an dem genannten Nagelschaft wenigstens eine Verbindungs-Querdurchbohrung derart angeordnet und aufgeweitet ausgebildet ist, daß sie ein zugehöriges stabförmiges Kalotten-Verbindungselement mit ausgeprägtem Justier-Bewegungsspiel von lateral her führt und aufnimmt.

Zweckmäßig kann an der Unterseite des Kopfkalotte eine Ausformung ausgebildet sein, die mit einem korrespondierenden, dem Nagelschaft zugeordneten Lagerteil, das heißt z.B. mit einem Adapterstück oder mit dem Nagelschaft direkt, zur Höhenverstellung der Kopfkalotte bewegbar verbunden ist.

In erfindungsgemäßer Ausgestaltung ist an der dem Nagelschaft zugewandten Unterseite der Kopfkalotte eine auf den proximalen Abschnitt des Nagelschafts aufsetzbare Ausnehmung ausgebildet, die in der der Erstreckungsrichtung des Nagelschafts entsprechenden Richtung durchgehend offen ist, wobei an der Kalotten-Anschlußseite im Bereich oder in der Nähe der Ausnehmung wenigstens eine Bohrung zur kraftschlüssigen Aufnahme des zugehörigen medialen Endes des Kalotten-Verbindungselements ausgebildet ist.

Besonders zweckmäßig kann zwischen dem Nagelschaft und der Kopfkalotte eine Schiebeverbindung zum relativen stufenlosen Schiebeversatz der Kopfkalotte längs des Nagelschafts ausgebildet werden. In bevorzugter Ausgestaltung weist die Schiebeverbindung wenigstens ein in der Erstreckungsrichtung des Nagelschafts entsprechender Richtung erweitertes Durchgangsloch auf, das jeweils eine Verbindungs-Querdurchbohrung bildet, in der ein zugeordnetes Kalotten-Verbindungselement in Loch-Erweiterungsrichtung stufenlos versetzbar ist.

Um die gesamte gewölbte Oberfläche der Kopfkalotte geschlossen auszubilden, wird in erfindungsgemäßer Ausgestaltung an der Unterseite der Kopfkalotte ein dort hervorstehendes Anschlußstück ausgebildet, in dem die Schlitzausnehmung vorgesehen wird. Wenngleich es denkbar ist, die Kopfkalotte durch Steckverbindung mit einem in die Verbindungs-Querdurchbohrung einfassenden Stift an dem Nagelschaft festzusetzen, wird in bevorzugter erfindungsgemäßer Gestaltung wenigstens eine die Kopfkalotte mit dem Nagelschaft fest verbindende Verbindungsschraube vorgesehen.

In bevorzugter Ausgestaltung der Erfindung wird wenigstens eine Verbindungs-Querdurchbohrung für ein zugehöriges Kalotten-Verbindungselement in dem mit dem Tuberculum majus des Humerus korrespondierenden proximalen Endbereich des Nagelschafts ausgebildet. Insbesondere in Kombination mit einer solchen Gestaltung kann der Nagelschaft in Verbindung mit wenigstem einer Knochen- und/oder Versorgungsschraube eingesetzt werden. Zu diesem Zweck kann wenigstens eine Querdurchbohrung eines zugehörigen Stabelements in einem Schaft-Zwischenabschnitt, der mit dem Halsbereich (Collum chirurgicum) des Humerus oder einem distalwärts daran angrenzenden Abschnitt korrespondiert, so angeordnet und ausgerichtet werden, daß das Versorgungs-Stabelement im medialen Bereich des Humeruskopfes zu liegen kommt.

Der erfindungsgemäß zum Herstellen der Osteosyntheseeinrichtung und/oder der Humeruskopfprothese vorgesehene Marknagelschaft kann zweckmäßig eine Gruppe von Querdurchbohrungen aufweisen, die derart angeordnet und ausgerichtet sind, daß zugehörige Versorgungsschrauben typische Muster kapitaler und subkapitaler Humerusfrakturen erfassen. Die Versorgungsschrauben sind dann vorteilhaft so orientiert, daß sie in dem medialen Bereich des Humeruskopfes zu liegen kommen. Um insbesondere Frakturen mit größerer Zahl von Bruchfragmenten zu versorgen, kann die Osteosyntheseeinrichtung in erfindungsgemäßer Ausgestaltung wenigstens ein Versorgungs-Stabelement umfassen, das so angeordnet und ausgerichtet ist, daß es sich in dem Humeruskopf nach dorsal und/oder ventral erstreckt. Bei dieser Ausgestaltung ist es von besonderem Vorteil, wenn für ein Paar oder eine Gruppe von Stabelementen wenigstens zwei Querdurchbohrungen nebeneinander längs des Marknagelschafts und sich unter spitzem Winkel kreuzend ausgebildet werden.

Besonders vorteilhaft und zweckmäßig kann in dem Nagelschaft der erfindungsgemäßen Einrichtungen wenigstens eine Verbindungs-Querdurchbohrung für das zugeordnete Kalotten-Verbindungselement so angeordnet und ausgerichtet sein, daß die Verbindungs-Querdurchbohrung wahlweise auch zur Aufnahme eines Versorgungs-Stabelements, vorzugsweise in Form einer Versorgungsschraube, nutzbar ist.

In bevorzugter erfindungsgemäßer Ausgestaltung weist der Nagelschaft eine proximale Stirnfläche auf, die sich bei in den Humerus eingebrachtem Nagelschaft auch bei angesetzter Kopfkalotte an der oberen proximalen Seite des Humerus in freiem operativem Zugriff befindet. Zweckmäßig wird in dem proximalen Schaftende ein Anschluß für ein Zielgerät ausgebildet. In weiterer vorteilhafter Gestaltung kann ein Verbindungsmittel, insbesondere eine Verbindungsschraube mit zugeordneten Bohrungen bzw. Löchern, in einer solchen Höhe am Nagelschaft angeordnet werden, daß der Nagelschaft mit seiner proximalen Stirnfläche unterhalb des Scheitelpunktes des Humeruskopfes bzw. der Kopfkalotte endet.

Eine andere besondere Ausgestaltung der Erfindung besteht darin, daß wenigstens ein Verbindungs-Adapterstück vorgesehen wird, das Nagelschaft und Kopfkalotte in Verbindung mit dem stabförmigen Verbindungselement derart miteinander verbindet, daß die Kopfkalotte wenigstens in einer Raumrichtung in ihrer Position relativ zu dem Nagelschaft verstellbar und feststellbar ist. Dabei ist es besonders zweckmäßig, die Kopfkalotte höhen-verschwenkbar an oder in dem Adapterstück zu lagern, während dieses seinerseits drehverschwenkbar um die Nagelschaft-Längsachse und/oder höhenverstellbar längs des Nagelschaftes an oder in diesem gelagert wird.

Gemäß weiterer erfindungsgemäßer Ausgestaltung für Bausatz und Humeruskopfprothese wird an der Unterseite der Kopfkalotte als Bestandteil eines Verbindungsmittels ein halsförmiges Ansatzstück zur klemmenden Verbindung mit dem Nagelschaft ausgebildet ist, wobei das Ansatzstück ein eine Ausnehmung bildendes Steckloch mit wenigstens einem lateralen Wandelement aufweist, das den Nagelschaft im Zustand der Steckverbindung lateral hintergreift. Vorzugsweise ist das Klemmelement mittels einer insbesondere in dem erfindungsgemäßen Bausatz enthaltenen Klemmschraube spannbar, die in das Ansatzstück im Bereich zwischen Kalottenunterseite und Klemm-Steckloch eingreift.

Unteransprüche sind auf die genannten und noch andere zweckmäßige und vorteilhafte Ausgestaltungen der Erfindung gerichtet. Besonders zweckmäßige und vorteilhafte Ausbildungsformen oder -möglichkeiten der Erfindung werden anhand der folgenden Beschreibung der in der schematischen Zeichnung dargestellten Ausführungsbeispiele beschrieben. Es zeigen
- Fig. 1 bis 3: in Ansicht von dorsal, proximal und lateral eine Osteosynthese- einrichtung, zusammengefügt aus Implantat-Einzelteilen eines er- findungsgemäßen Bausatzes,
- Fig. 4 bis 7: in Ansicht von dorsal, proximal und lateral, in implantierter Anord- nung und in Explosionsdarstellung, eine Humeruskopfprothese, zusammengesetzt aus Implantat-Einzel- teilen eines erfindungsgemäßen Bausatzes,
- Fig. 8 bis 11: in Ansicht von dorsal, proximal und lateral, in Explosionsdarstel- lung und in implantierter Anordnung, eine Humeruskopfprothese, zusammengesetzt aus Implantat-Einzel- teilen eines erfindungsgemäßen Bausatzes,
- Fig. 12 bis 14: in Ansicht von dorsal und proximal, in Explosionsdarstellung und in implantierter Anordnung, eine Humeruskopf- prothese, zusammengesetzt aus Implantat-Einzelteilen eines erfin- dungsgemäßen Bausatzes, und
- Fig. 15 und 16: in Ansicht von ventral sowie im Schnitt A-A eine Humeruskopfprothese, zusammengesetzt aus Implatat-Ein- zelteilen eines erfindungsgemäßen Bausatzes.

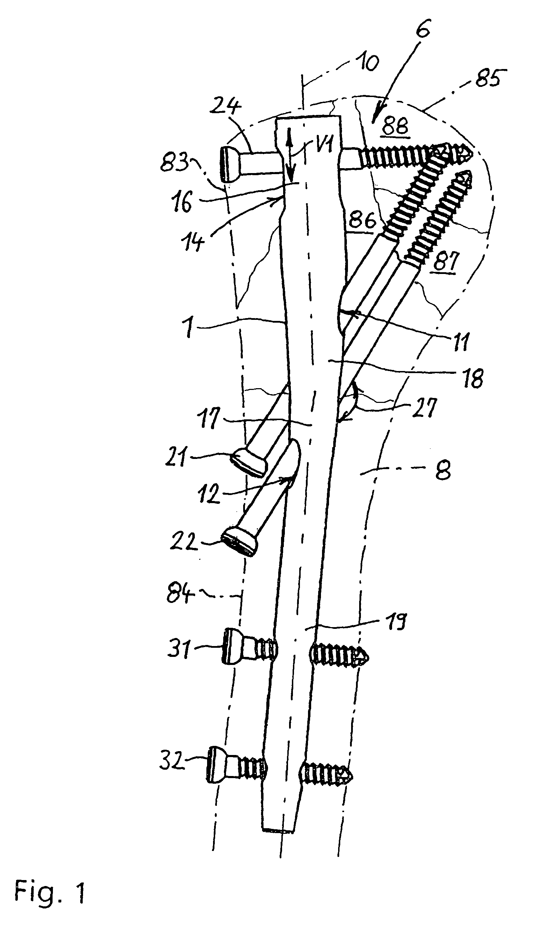

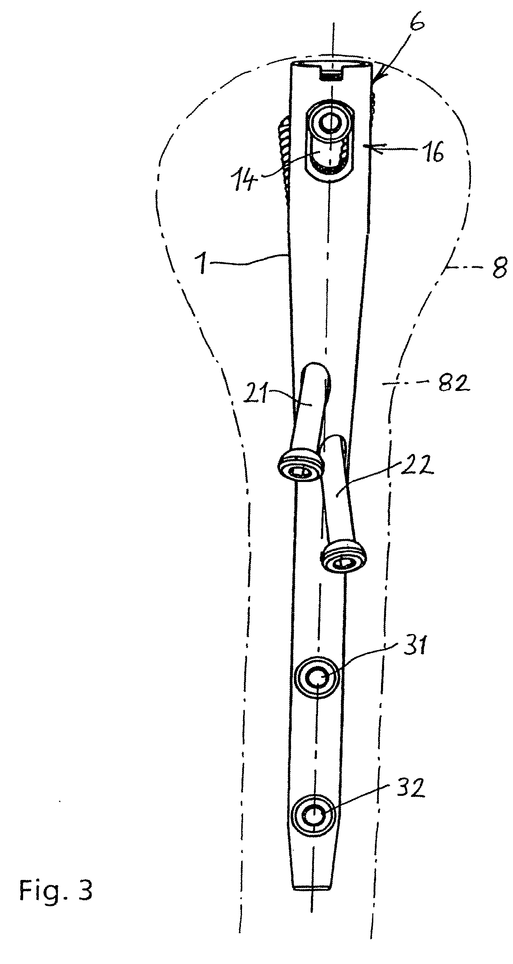

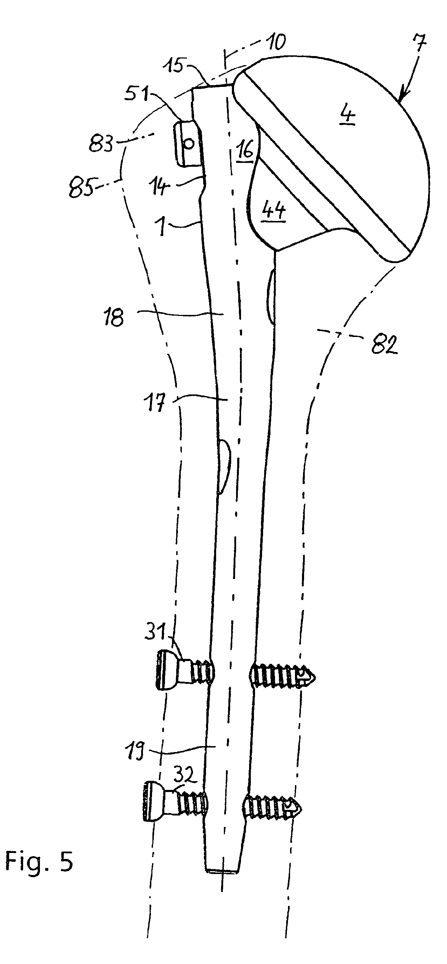

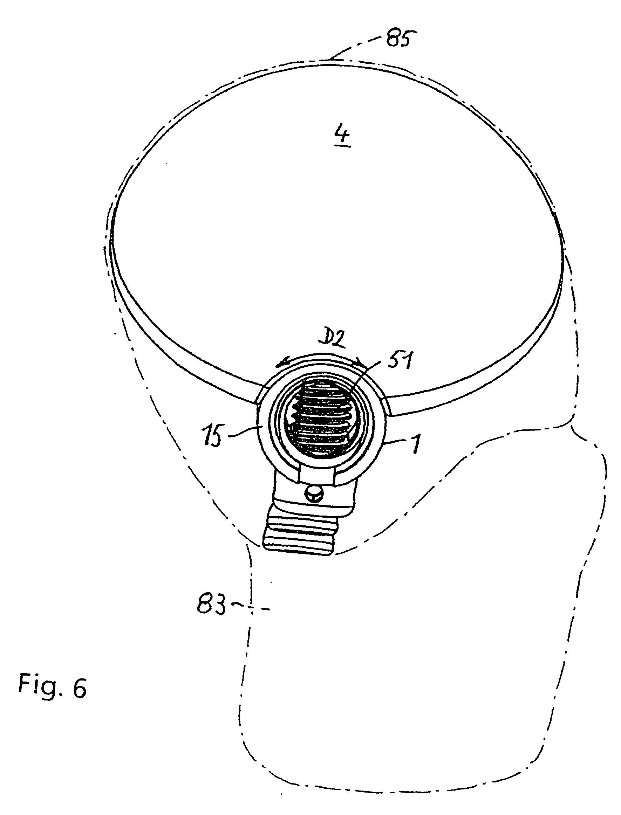

In Fig. 1 bis 7 sind im Ausführungsbeispiel eine Osteosyntheseeinrichtung 6 und eine Humeruskopfprothese 7 dargestellt, die aus Teilen eines zugehörigen erfindungsgemäßen Bausatzes zusammengefügt werden.

Der Bausatz umfaßt als zentrales Einzelteil einen von proximal in die Markhöhle des Humerus 8 einführbaren, mit seiner Längsachse 10 sich lang erstreckenden hohlen Nagelschaft 1, der, wie ersichtlich, eine einteilige und einstückige Basiseinheit bildet. Der Nagelschaft 1 weist Kreisquerschnitt auf, der im unteren distalen Abschnitt kleiner als im oberen proximalen Abschnitt ist. Das distale Schaftende ist spitz eingezogen, um Weichteilirritation zu verhindern. Ausgehend von seinem dem Hals (Collum chirurgicum) 82 entsprechenden Schaftabschnitt 18 erstreckt sich der Nagelschaft 1 zu seinem proximalen Endabschnitt 16 hin entsprechend der lateralwärts gerichteten Krümmung des Humerus 8. Der proximale Endabschnitt 16 des Schafts 1 ist mit üblicher Innenbohrung zum Anschluß eines Zielgeräts ausgestattet. In einem Schaft-Zwischenabschnitt 17, der mit dem chirurgischen Halsbereich (Collum chirurgicum) 82 des Humerus 8 und einem distalwärts daran angrenzenden Abschnitt korrespondiert, sind für Versorgungsschrauben 21, 22 von lateral nach medial sich erstreckende Querdurchbohrungen 11, 12 ausgebildet, die benachbart und längs des Nagelschafts 1 nebeneinander angeordnet sind. Sie sind von unten nach oben auf den medialen Bereich des Humeruskopfes 85 ausgerichtet, und zwar mit zur Längsachse 10 relativ steilen Winkeln 27 von z.B. ca. 140°. Im oberen proximalen Endabschnitt 16 des Nagelschafts 1 ist eine von lateral nach medial sich erstreckende Querdurchbohrung 14 wahlweise für eine Versorgungsschraube 24 und eine ein stabförmiges Verbindungselement bildende Kalotten-Verbindungsschraube 51 ausgebildet. Die Querdurchbohrung 14 ist in dem mit dem Tuberculum majus 83 des Humerus 8 korrespondieren proximalen Endbereich des Nagelschafts 1 angeordnet. Die Querdurchbohrung 14 ist so ausgerichtet, daß die Schrauben 51 bzw. 24 jedenfalls im wesentlichen in der Humerus-Frontalmittelebene (entsprechend der AP-Ebene) 800 zu liegen kommen.

Die Achsen der Querdurchbohrungen 11 und 12 sind mit spitzen Winkeln 25 von z. B. 15° aus der Frontalmittelebene 800 herausgekippt, so daß sich die von den Querdurchbohrungen 11, 12 aufgenommenen und geführten Versorgungsschrauben 21, 22 nebeneinander und kreuzend unter spitzem Winkel 26 von ca. 30° erstrecken. Dabei kommt die Versorgungsschraube 21 mit diagonaler Richtungskomponente nach dorsal und die Versorgungsschraube 22 mit diagonaler Richtungskomponente nach ventral in dem Humeruskopf 85 zu liegen. Im distalen Fußabschnitt 19 des Nagelschalfts 1 sind Querdurchbohrungen zur Aufnahme von Verriegelungsschrauben 31, 32 vorgesehen.

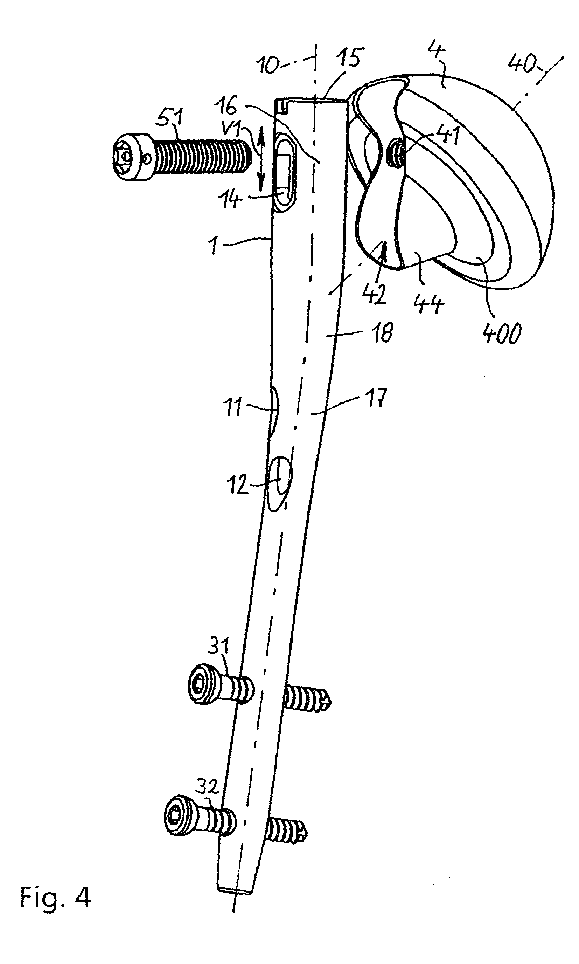

Der erfindungsgemäße Bausatz umfaßt weiterhin Kopfkalotten, die künstliche Humerus-Gelenkköpfe unterschiedlicher Größe bilden. Im Ausführungsbeispiel der Fig. 4 bis 7 ist eine Kopfkalotte 4 vorgesehen, die so bemessen ist, daß sie den im Beispiel dargestellten zerstörten Humeruskopf 85 ersetzt. Als weitere Einzelteile des Bausatzes sind die Verriegelungsschrauben 31, 32, die Verbindungsschraube 51 sowie die Versorgungsschrauben 21, 22 und 24 vorgesehen. Die genannten Schrauben sind zweckmäßig in unterschiedlichen Größen in dem Bausatz enthalten, um eine Auswahl für Oberarmknochen unterschiedlicher Größe zur Verfügung zu haben. Auch enthält der Bausatz in Anpassung auf Oberarmknochen unterschiedlicher Größe zweckmäßig eine Auswahl von unterschiedlichen Nagelschäften 1 mit entsprechenden Schrauben.

Gemäß Fig. 1 bis 3 ist aus Teilen des erfindungsgemäßen Bausatzes die Osteosyntheseeinrichtung 6 zur Verbindung der Endstellen von Kopffrakturteilen 86, 87 und 88 zusammengesetzt worden. Die beiden nebeneinander angeordneten Querdurchbohrungen 11, 12 führen die Versorgungsschrauben 21, 22, wobei sie passend in den Bohrungen 11, 12 sitzen. An ihren medialen Enden sind die Versorgungsschrauben 21, 22 mit selbstschneidendem Gewinde versehen.

Im oberen Bereich des Oberarmknochens ist die Versorgungsschraube 24 gesetzt worden, die von lateral durch den Tuberculum majus 83 eingebracht wird und in den oberen medialen Teil des Humeruskopfes 85 mit selbstschneidendem Gewinde einfaßt. Die Versorgungsschraube 24 verbindet die Endstellen der Frakturfragmente 86 und 88 miteinander. Die Versorgungsschraube 24 ist etwa im Winkel von 90° zur Achse 10 im Bereich des Schaftabschnitts 16 ausgerichtet, wobei ihre Achse zumindest im wesentlichen in der Humeruskopf-Frontalmittelebene 800 zu liegen kommt. Insoweit befindet sich die Versorgungsschraube 24 passend im Sitz zwischen Wänden der Querdurchbohrung 14. Die Querdurchbohrung 14 ist längs der Achse 10 in Form eines rechteck- oder ovalförmigen Langlochs in Längs- und Umfangsrichtung des Nagelschafts 1 erweitert, so daß die Versorgungsschraube 24 in Anpassung auf die räumliche Lage der Frakturfragmente in Verstellrichtung V1 stufenlos höhenversetzt und in korrekter Position festgesetzt werden kann.

Wie aus Fig. 4 bis 7 ersichtlich, wird der für die Osteosyntheseeinrichtung 6 der Fig. 1 bis 3 verwendete Nagelschaft 1 wahlweise zum Aufbau einer Humeruskopfprothese 7 verwendet. Diese umfaßt die Kopfkalotte 4, den Nagelschaft 1 und als Verbindungsmittel die diese beiden Teile fest miteinander verbindende Verbindungsschraube 51.

Die Kopfkalotte 4 weist eine dem Nagelschaft 1 zugewandte Anschluß- oder Unterseite 400 auf, an der eine Ausformung vorgesehen ist. Diese ist durch ein halsförmiges, zur freien Seite hin sich verjüngendes, mundförmiges Anschlußstück 44 gebildet. Die Kopfkalotte 4 ist mit dem Hals-Anschlußstück 44 einstückig. Die Längsachse des Anschlußstückes 44 erstreckt sich zumindest im wesentlichen koaxial mit der Haupt- und Symmetrieachse 40 der Kopfkalotte 4.

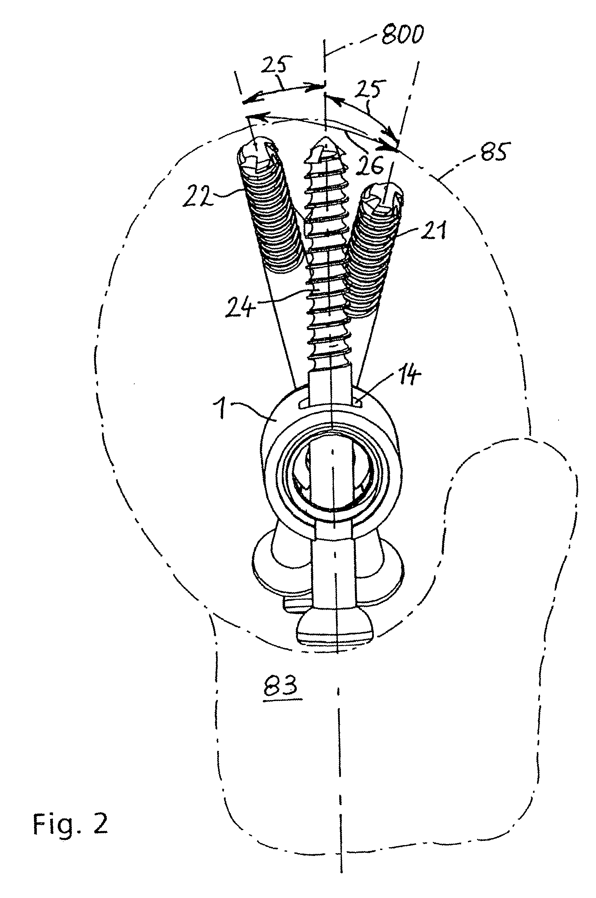

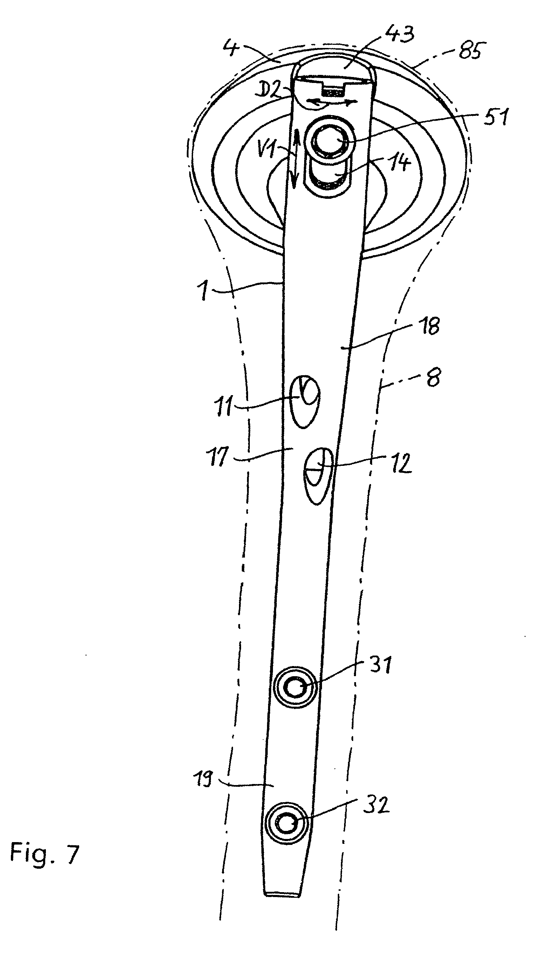

In dem Anschlußstück 44 ist eine Schlitzausnehmung ausgebildet, die in Richtung parallel zur Längsachse 10 des Nagelschafts 1 und nach lateral durchgehend offen ist. Der Schlitz erstreckt sich in der Frontalmittelebene 800 des Humeruskopfes 85. Ebenfalls im Bereich dieser Ebene 800 ist am Boden der Schlitzausnehmung eine Gewindebohrung 41 eingearbeitet, in die das mit Feingewinde ausgestattete mediale Ende der Verbindungsschraube 51 geschraubt wird. Der Schlitz bildet eine eine Führung bildende Sitzausnehmung 42, mittels derer die Kopfkalotte 4 im Schaftabschnitt 16 an der medialen Seite des Nagelschafts 1 auf diesem aufsitzt. In Kombination mit der Langloch-Querdurchbohrung 14 ist zwischen dem Nagelschaft 1 und der Kopfkalotte 4 eine ausgeprägtes Bewegungsspiel gewährleistende Schiebeverbindung ausgebildet, um die Kopfkalotte 4 in Richtung V1 in der Höhe zu verstellen und zu justieren, bevor sie mit der Schraube 51 in gewünschter Schafthöhe befestigt wird. Man erkennt, daß vor dem Festziehen der Schraube 51 bereits eine unverlierbare Verbindung zwischen der Kopfkalotte 4 und dem Nagelschaft 1 hergestellt ist. Der Nagelschaft 1 endet mit seiner proximalen Stirnfläche 15 unterhalb des Scheitelpunktes der Kopfkalotte 4. Man erkennt, daß die Nagel-Stirnfläche 15 frei von der angesetzten Kopfkalotte 4 und damit in operativem Zugriff insbesondere zur Verbindung mit einem Zielgerät bleibt. Wie insbesondere aus Fig. 2 und 7 ersichtlich, ist die Querdurchbohrung 14 auch in Umfangsrichtung D2 des Schafts 1 aufgeweitet, so daß die Kopfkalotte 4 zum justierenden Ausrichten auch in dieser Richtung im Aufsitz auf dem Nagelschaft 1 drehbeweglich ist, bevor die Schraube 51 festgezogen wird. Beim Befestigen der Kopfkalotte 4 greift der Kopf der Schraube 51 lateral an dem Nagelschaft 1 an.

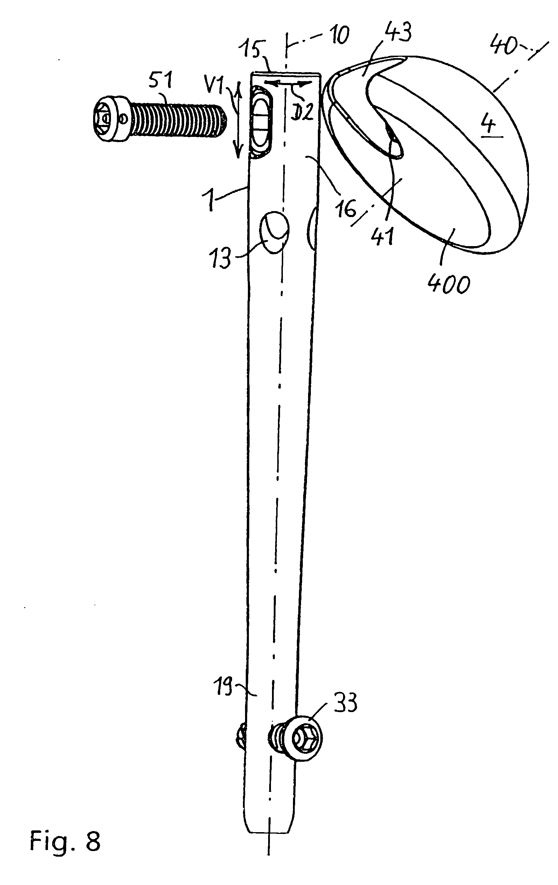

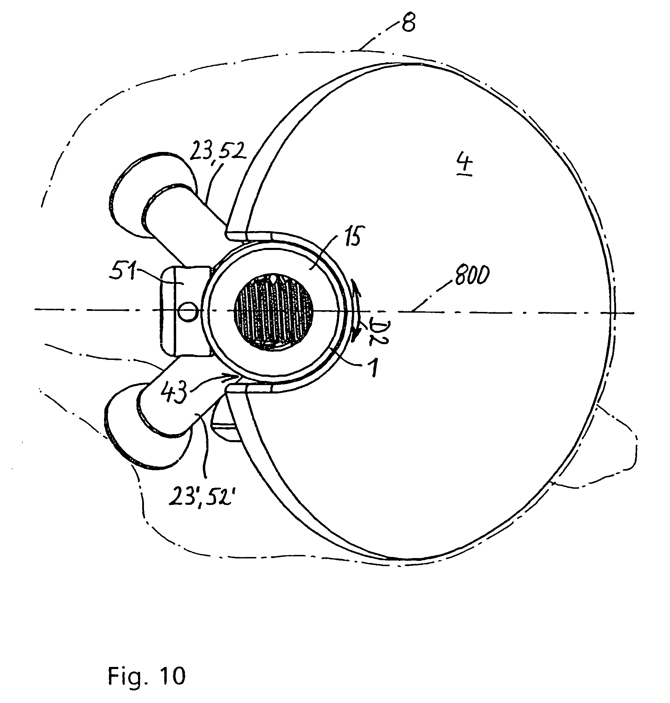

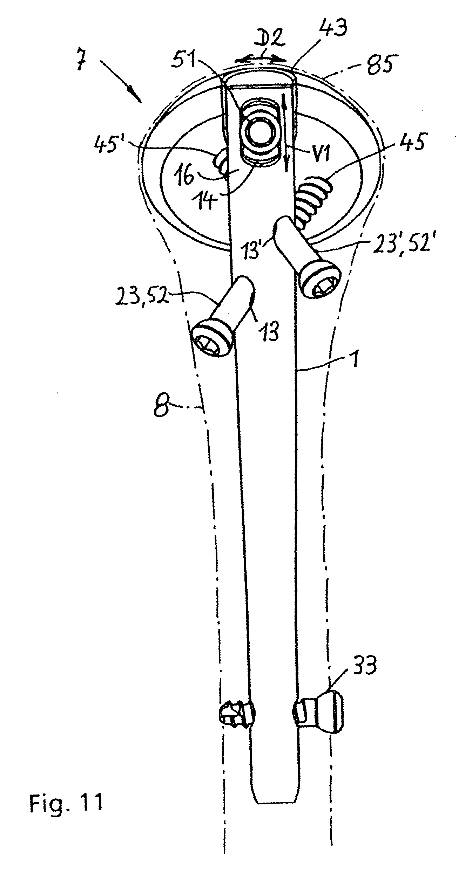

Anhand der Fig. 8 bis 11 ist eine erfindungsgemäße Humeruskopfprothese 7 dargestellt, die einen Nagelschaft 1, eine Kopfkalotte 4, eine diese beiden Teile verbindende Verbindungsschraube 51 sowie zusätzlich zwei einander benachbarte, sich unter spitzem Winkel kreuzende Versorgungsschrauben 23, 23' umfaßt, die gegebenenfalls zusätzlich Verbindungsschrauben 52, 52' bilden. Bei diesem Ausführungsbeispiel ist als Ausformung 43 ein nach lateral offener Durchgangsschlitz unmittelbar zwischen der gewölbten Oberseite und der flachen, die Anschlußseite bildenden Unterseite 400 der Kalotte 4 ausgebildet. Infolgedessen kommt das proximale Ende des Nagelschafts 1 in dem lateralen oberen Bereich der Kalotte 4 zu sitzen, wobei die Stirnfläche 15 des Nagelschafts 1 durch den Schlitz von oben frei zugänglich bleibt. Eine Gewindebohrung 41 für die Verbindungsschraube 51 befindet sich im unteren (distalen) Bodenabschnitt des Schlitzes. Wie bei dem Ausführungsbeispiel der Fig. 4 bis 7 ist zwischen der Kalotte 4 und dem Nagelschaft 1 eine formschlüssige Schiebeverbindung hergestellt, wobei die Verbindungsschraube 51 zum Ausrichten der Kopfkalotte 4 in dem Langloch 14 in Umfangsrichtung D2 und in Richtung V1 längs des Nagelschafts 1 schiebeversetzbar ist.

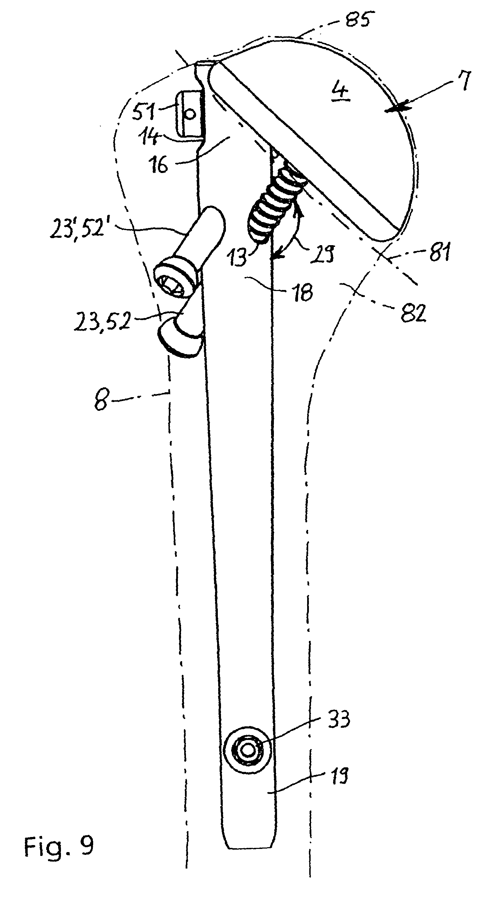

In Fig. 8 wird eine Kalotte 4 verwendet, die allein mit der einzigen zum Ausrichten und Justieren vorgesehenen Verbindungsschraube 51 an dem Nagelschaft 1 befestigt wird. Hingegen wird gemäß Fig. 9 bis 11 eine Kopfkalotte 4 mit zusätzlichen Befestigungsbohrungen 45, 45' verwendet. Den beiden Schrauben 23, 52 und 23', 52' kommt dann eine Doppelfunktion zu. Sie sind jeweils in Passung in zugeordneter Querdurchbohrung 13, 13' geführt. Die Bohrungen 13, 13' kommen im Bereich des Humerushalses (Collum chirurgicum) 82 diagonal zu liegen, wobei sie von lateral nach medial zur Unterseite der Kopfkalotte 4 bzw. zum Hals des Oberarmknochens (Collum anatomicum) 81 schräg nach oben gerichtet sind. Sie sind aus der Frontalmittelebene 800 mit einem Winkel 29 von ca. 45° herausgekippt, so daß sie dorsal bzw. ventral gerichtet sind. Die Schrauben 23, 52 und 23', 52' werden von lateral in die Schräg-Querdurchbohrung 13, 13' eingeführt. Jede Schraube ist einerseits als Knochenschraube 23, 23' ausgebildet, die den Knochenbereich zwischen der Kopfkalotte 4 und dem Nagelschaft 1 durchsetzt. Sie kann dort als Fraktur-Versorgungsschraube genutzt werden. Andererseits bildet jede Schraube eine zusätzliche Verbindungsschraube 52, 52', die mit ihrem medialen Ende in die zugeordnete Gewindebohrung 45, 45' an der Unterseite der Kopfkalotte 4 kraftschlüssig eingedreht wird.

In abgewandelter Ausführung kann die obere Verbindungsschraube 51 auch entfallen, und die Kopfkalotte 4 kann mit der Schraube 23, 52 und/oder der Schraube 23', 52' fest an dem Nagelschaft 1 befestigt werden.

Der Nagelschaft 1 wird im Ausführungsbeispiel der Fig. 7 bis 10 mit einer distalen Verriegelungsschraube 33 verriegelt. Der Nagelschaft 1 weist Kreisquerschnitt auf, der zum distalen Ende hin abnimmt. Das distale Schaftende ist zur Verhinderung von Weichteilirritation abgeschrägt ausgebildet.

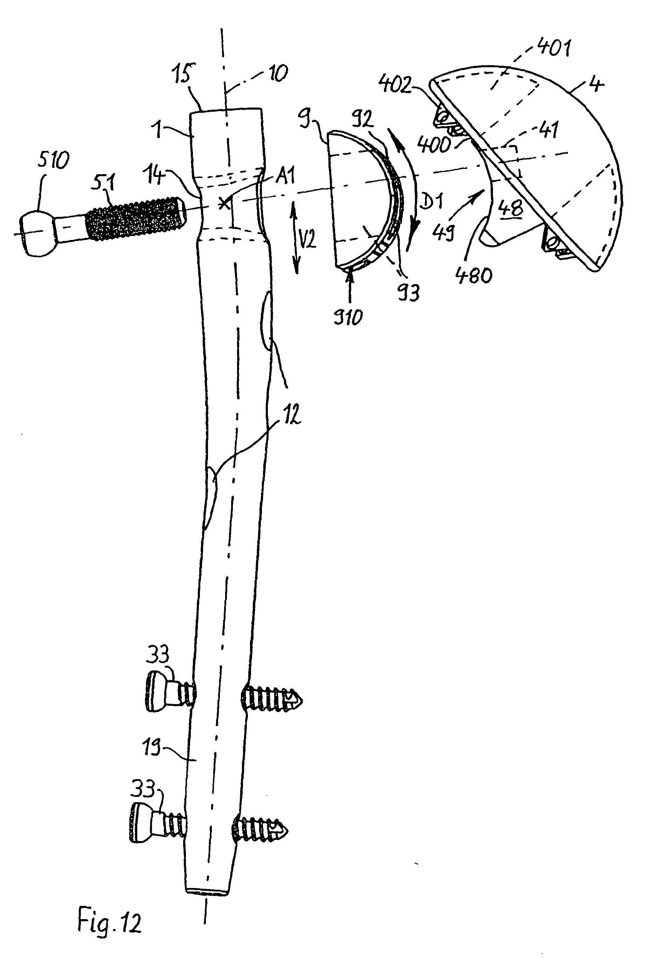

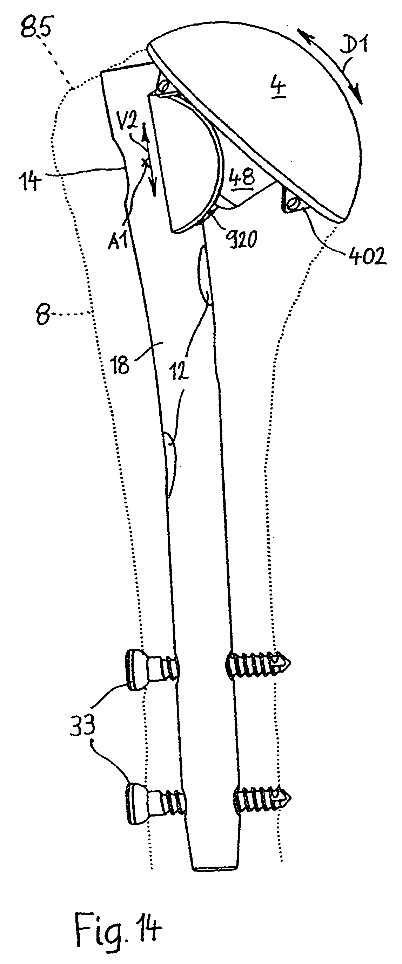

Die Figuren 12 bis 14 zeigen Ausführungsformen einer Humeruskopfprothese des erfindungsgemäßen Bausatzes mit einem Adapterstück 9 zwischen einem Nagelschaft 1 und einer Kopfkalotte 4. Für Teile der Prothese, die denen der zuvor beschriebenen Ausführungsformen entsprechen, werden gleiche Bezugszeichen verwendet, und insoweit wird auf die vorstehende Beschreibung Bezug genommen.

Das Adapterstück 9 verbindet den Nagelschaft 1 mit der Kopfkalotte 4 derart, daß die Kopfkalotte 4 dreidimensional in ihrer Position relativ zu dem Nagelschaft 1 einstellbar ist.

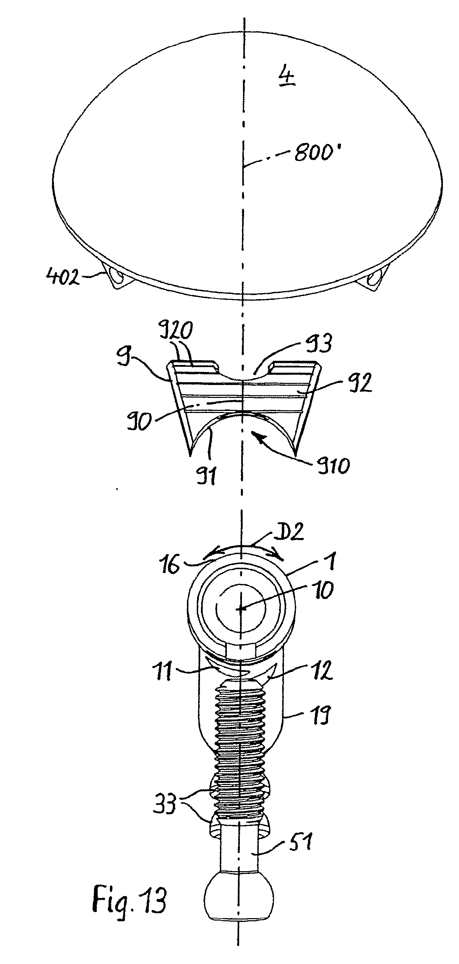

Das Verbindungs-Adapterstück 9 ist als separates Gelenkstück ausgebildet und weist in seiner Grundform im wesentlichen die Form eines Zylinderabschnitts auf, wie dies insbesondere aus Fig. 13 deutlich wird. Die kreisförmig gewölbte Zylinder-Abschnittsfläche des Adapterstücks 9 bildet eine konvexe Lagerfläche 92 einer Führungslagerung für die Kopfkalotte 4. Wie man insbesondere der Ansicht in Fig. 13 entnimmt, fällt die Symmetrieebene 90 des Adapterstück-Zylinderabschnitts jedenfalls im wesentlichen mit der der Frontalmittelebene (AT-Ebene) entsprechenden Ebene 800' zusammen. Die Lagerfläche 92 ist der Kopfkalotte 4 zugewandt. An der der Zylinderabschnittsfläche abgewandten Seite des Adapterstücks 9 ist eine halbkreisförmige Ausnehmung 910 ausgebildet, die parallel zu der Längsachse 10 des Nagelschafts 1 durchgehend offen ist.

In dem Adapterstück 9 ist ein Durchgangsloch 93 für die Verbindungsschraube 51 ausgebildet, das mittig durch den Zylinderabschnitts-Körper hindurchgeht und dessen Achse in der Körper-Symmetrieebene 90 liegt. Das Durchgangsloch 93 ist nach oben und nach unten, das heißt in Richtung längs des Nagelschafts 1 und in der Symmetrieebene 90 spaltförmig erweitert und so groß bemessen, daß die Verbindungsschraube 51 nach oben und nach unten versetzt und/oder verschwenkt werden kann.

Die Kopfkalotte 4 weist an ihrer Unterseite 400 ein durch eine Ausformung gebildetes hervorstehendes Anschlußstück 48 auf, das mit einer zu der Zylinderabschnitts-Lagerfläche 92 korrespondierenden konkaven Lagerfläche 480 einer Ausnehmung 49 ausgeformt ist.

Im montierten Zustand sitzt das Adapterstück 9 mit seiner Lagerfläche 91 auf der korrespondierenden Zylinderfläche des Nagelschafts 1 auf, und die Kopfkalotte 4 sitzt mit ihrer Lagerfläche 480 auf der Zylinderabschnitts-Lagerfläche 92 auf. Die Verbindungsschraube 51 durchgreift die Langlochbohrung 14 in dem Nagelschaft 10 und das Durchgangsloch 93 in dem Adapterstück 9 und faßt mit ihrem Gewinde in die Gewindebohrung 41 an der Unterseite der Kopfkalotte 4 ein, wobei das Loch 41 in den Boden der Lagerausnehmung 49 eingebracht ist.

Solange die Verbindungsschraube 51 noch in bereits unverlierbarer Verbindung nicht fest angezogen ist, ist die Kopfkalotte 4 in ihrer Höhenposition längs des Nagelschafts 1 mit Freiheitsgraden V2 und D1 stufenlos höhen- und winkelverstellbar. Dabei ist die Kalotte 4 um die Längsachse 10 des Nagelschafts 1 stufenlos schwenkbar gelagert und infolgedessen mit einem weiteren Freiheitsgrad D2 winkelverstellbar. Dies gelingt dadurch, daß einerseits das Adapterstück 9 längs des Nagelschafts 1 höhenverstellbar sowie um seine Achse 10 schwenkbar auf diesem aufsitzt und daß andererseits die Kopfkalotte 4 um eine gedachte, zu dem Zylinderabschnitt zugehörige Zylinderachse A1 schwenkbar auf dem Adapterstück 9 aufsitzt, wobei in den Bohrungen 14 und 93 ausreichend Bewegungsraum für die noch nicht festgezogene Verbindungsschraube 51 vorgesehen ist. Durchaus kann zum Beispiel und zweckmäßig das Verstellmaß in Richtung D2 in der Größenordnung von 20°, in Richtung D1 in der Größenordnung von 15° und in Richtung V2 linear in der Größenordnung von 5 mm liegen. Nachdem die Kopfkalotte 4 ausgerichtet worden ist, wird die Verbindungsschraube 51 angezogen, um die eingestellte Position festzusetzen. Der Schraubenkopf 510 greift an der lateralen Seite des Nagelschafts 1 an.

Zweckmäßig weist die Lagerfläche 92 des Adapterstücks 9 eine Struktur 920 auf. Diese dient sowohl der Anlageverbesserung als auch einer Verbesserung der Verstellbewegung der intraoperativ zu manipulierenden Implantatteile. Im Ausführungsbeispiel ist eine Riffelung mit über die Höhe des Zylinderabschnitts sich erstreckenden , durch vertiefte Linienbereiche getrennten Streifenabschnitten ausgebildet.

Wie in Fig. 12 dargestellt, kann es zweckmäßig sein, daß die Querdurchbohrung 14 nach medial konisch erweitert wird. Man erreicht dadurch, daß die Verbindungschraube 51 - im noch nicht angezogenen Zustand - um ihren Schraubenkopf 510, der im engen Bereich der konischen Bohrung zu liegen kommt, geführt schwenkbewegbar ist, nämlich einerseits in Richtung D1 und andererseits in Richtung D2. Zu diesem Zweck wird der Zylinderabschnitts-Körper des Adapterstücks 9 so dimensioniert, daß die Achse A 1 in dem Bereich des Schraubenkopfes 510 der eingedrehten Schraube 51 zu liegen kommt. Zudem beläßt man im engen Bereich der konischen Bohrung noch genügend Spiel, um die Schiebeverstellung V2 zu gewährleisten.

Eine Maßnahme im Rahmen der Erfindung kann insbesondere auch darin bestehen, das Adapterstück 9 in unveränderbarer Höhenposition an dem Nagelschaft 1 zu lagern, die Verstellmöglichkeit in Richtung V2 also zu beseitigen. Zweckmäßig kann das Adapterstück dann in einer Ausnehmung in dem Nagelschaft 1 schwenkbar um dessen Längsachse 10 gelagert werden. Weiterhin kann ein Adapterstück gemäß der Erfindung auch unverstellbar an dem Nagelschaft 1 festgesetzt oder einteilig mit diesem ausgebildet sein. Die Kopfkalotte bleibt dann über die durch den ausreichend bemessenen Spalt 93 in dem Adapter sich erstreckende Verbindungsschraube 51 längs der Führungsfläche 92, die auch sphärisch ausgebildet sein kann, höhen- bzw. winkelverstellbar.

Die Kopfkalotte 4 des Ausführungsbeispiels gemäß Fig. 12 bis 14 ist außerhalb des an ihrer Unterseite hervorstehenden Anschlußstückes 48 im Bereich 401 hohl ausgebildet, so daß ihr Gewicht reduziert ist. Die erfindungsgemäße Verstell-Verbindung begünstigt diese Konstruktion. Am Rand der Kalottenunterseite sind Ösen 402 zur Fragmentbefestigung angeordnet.

Die Teile der Einrichtungen gemäß Fig. 1 bis 7, 8 bis 11 und/oder 12 bis 14 sind zweckmäßig gemeinsame Bestandteile eines erfindungsgemäßen Bausatzes. Insbesondere können auch mehrere, unterschiedlich dimensionierte Adapterstücke 9 in einem Bausatz bereitgehalten werden. Von besonderem Vorteil ist aber auch, daß mit ein und demselben Adapterstück 9 Kopfkalotten 4 unterschiedlicher Größe verwendbar sind, deren Ansatzstücke 48 im Bereich der Ausnehmung 49 einheitlich ausgebildet werden.

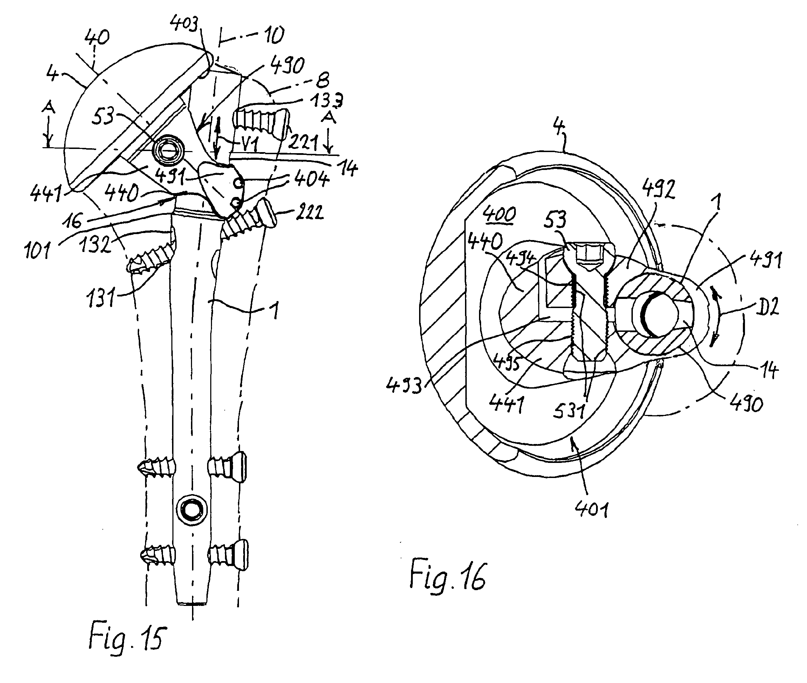

Die Fig. 15 und 16 zeigen eine weitere Ausführungsform einer erfindungsgemäßen Humeruskopfprothese. Für Teile, die denen der zuvor beschriebenen Ausführungsformen entsprechen, werden gleiche Bezugszeichen verwendet, und insoweit wird auf die vorstehende Beschreibung Bezug genommen.

An der Unterseite 400 einer Kopfkalotte 4 ist ein einen Bestandteil eines Verbindungsmittels bildendes halsförmiges Ansatzstück 440 ausgebildet. Ansatzstück 440 und Kopfkalotte 4 bilden eine einstückige Einheit. Das Ansatzstück 440 weist ein durchgehendes Steckloch 490 auf. Durch dieses Steckloch 490 wird ein Versorgungs-Nagelschaft 1 mit seinem proximalen Ende hindurchgesteckt. Die distale Seite des Ansatzstückes 440 bzw. der distale Rand des Steckloches 490 kommen gegen einen Vorsprung 101 in Form einer umlaufenden Schulter am Nagelschaft 1 zum Anschlag. Um die Prothese gewichtsmäßig möglichst leicht auszuführen, ist die Kopfkalotte 4, wie aus Fig. 16 ersichtlich, zum großen Teil hohl ausgebildet.

Ein laterales Wandelement 491 des Ansatzstückes 440 umgreift und hintergreift damit den Nagelschaft 1. Das Wandelement 491 bildet also einen Steg, der bei festgezogener Klemm-Verbindungsschraube 53 lateral an dem proximalen Abschnitt 16 des Nagelschafts 1 angreift. An dem Steg sind zwei Fixierungsösen 404 ausgebildet, um eine Cerclage mit Knochenfragmenten herstellen zu können. Das laterale Wandelement 491 geht einstückig in ein Wandelement 492 über, so daß ein bügelartiges Federteil ausgebildet ist, das bewegbar in eine Ausnehmung 493 des Ansatzstückes 440 eingreift. Das Feder-Wandelement 492 ist mittels einer Verbindungsschraube 53 gegen ein mit der Kopfkalotte 4 festes Wandelement 441 des Ansatzstückes 440 spannbar. Die Schraube 53 sitzt mit ihrem oberen Teil in einem Durchgangs-Gewindeloch 494 in dem Wandelement 492 und greift in ein Durchgangs-Gewindeloch 495 in dem festen Wandelement 441 ein.

Die mit Innengewinde 531 fluchtend ausgerichteten Durchgangslöcher 494, 495 sind beide dorsal und ventral mit einem Senkloch zur Aufnahme des Kopfes der Verbindungsschraube 51 ausgebildet. Man erreicht dadurch, daß die Verbindungsschraube 53 wahlweise von dorsal oder ventral gesetzt werden kann.

Das Ansatzstück 440 bildet mit den Wandelementen 441, 491 und 492 ein einstückiges Klemmteil, das durch Eindrehen und Anziehen der Verbindungsschraube 53 an dem proximalen Abschnitt 16 des Nagelschafts 1 fixiert wird. Zum Ausrichten der Kopfkalotte 4 kann diese in Richtung D2 um die Längsachse 10 des proximalen Nagelschafts 1 gedreht sowie längs dieses Schaftabschnitts 16 in Schieberichtung V 1 verschoben werden, bevor die Kopfkalotte 4 in der Klemmverbindung fixiert wird.

Mit der noch nicht fixierten Steckverbindung wird gezielt Bewegungsspiel zum Ausrichten und Justieren der Kopfkalotte 4 vorgegeben. Dabei sind die Kopfkalotte 4 und der Nagelschaft 1 in weitem Maß in Richtung V1 und in der Drehverbindung lose unverlierbar miteinander verbunden. In Schieberichtung V1 bildet der Vorsprung 101 einen distalen Anschlag, um für die Kalotte 4 eine Aufsteckbegrenzung zu erhalten.

Das Durchgangsloch 490 ist zur Haupt- und Symmetrieachse 40 der Kopfkalotte 4 derart von proximal nach distal gerichtet, daß ein spitzer Winkel zwischen der proximalen Nagelschaftachse 10 und der Kalottenachse 40 für die Neigung der Kopfkalotte 4 gegenüber dem Nagelschaft 1 bestimmt wird. Zweckmäßig wird der Querschnitt des Durchgangsloches 490 zumindest im wesentlichen in Übereinstimmung mit dem Querschnitt am proximalen Schaftabschnitt 16 vorgesehen, um eine geeignete Passung zu erhalten. Denkbar ist es allerdings auch, ein gewisses Querschnittsspiel des proximalen Nagelschafts 1 in einem Durchgangsloch mit ebenen Wänden od. dgl. zu belassen, um den Neigungswinkel zwischen der Kopfkalotte 4 und dem Nagelschaft 1 justieren zu können.

Die Teile der Einrichtungen gemäß Fig. 1 bis 7, 8 bis 11, 12 bis 14 und/oder 15, 16 sind zweckmäßig gemeinsame Bestandteile eines erfindungsgemäßen Bausatzes. Insbesondere erkennt man in Fig. 15, 16, daß der Nagelschaft 1 eine Querdurchbohrung 14 aufweist, um erfindungsgemäß zum Beispiel Kopfkalotten 4 entsprechend den Ausführungsbeispielen Fig. 8 bis 11 und/oder 12 bis 14 anzubringen. Im Ausführungsbeispiel gemäß Fig. 15, 16 weist der Nagelschaft 1 ein Paar Querdurchbohrungen 131, 132 etwa in der Höhe des chirurgischen Humushalses sowie eine Querdurchbohrung 133 im proximalen Endbereich des Nagelschafts 1 auf. Die Querdurchbohrungen 131, 132 sind ähnlich wie die Querdurchbohrungen 13, 13' im Ausführungsbeispiel der Fig. 8 bis 11 sich kreuzend angeordnet. Es handelt sich um Querdurchbohrungen für Schrauben zum Versorgen von Frakturen im Humerus-Kopfbereich, also für kapitale und subkapitale Frakturen. Wird ein solcher Versorgungsnagel wie in Fig. 15, 16 mit der Kopfkalotte 4 verbunden, so lassen sich die Bohrungen 131 bis 133 zum Setzen von Knochenschrauben 221, 222 nutzen.

## Patentansprüche

1. Bausatz für die Therapie von kapitalen und subkapitalen Humerusfrakturen (86-88), umfassend folgende zueinander gehörige Implantat-Einzelteile:
- Wenigstens einen von proximal in die Markhöhle des Humerus (8) einführbaren, mit seiner Längsachse (10) sich lang erstreckenden Nagelschaft (1), der wenigstens eine Versorgungs-Querdurchbohrung (11-14, 131-133) für wenigstens eine zugehörige Versorgungsschraube (21-24) zum Versorgen kapitaler und/oder subkapitaler Humerusfrakturen (86-88) aufweist;
- wenigstens eine an dem proximalen Ende des Nagelschafts (1) befestigbare, mittels Ausformung (42, 43, 49, 490) gegen den Nagelschaft (1) setzbare, als Humerus-Prothesengelenkkopf ausgebildete Kopfkalotte (4);
- wenigstens ein Verbindungsmittel zum Verbinden jeder Kopfkalotte mit dem Nagelschaft (1) und
- wenigstens eine Versorgungsschraube (21-24) und wenigstens eine Verriegelungsschraube (31-33),
wobei der genannte Nagelschaft (1), der augeteilter Bestandteil einer intramedullären Osteosyntheseeinrichtung (6) ist, die den Nagelschaft (1)*,* die wenigstens eine Versorgungsschraube (21-24) und wenigstens eine Verriegelungschraube (31-33) umfasst,
an seinem proximalen Ende im Bereich wenigstens einer genannten Versorgungs-Querdurchbohrung (11-14, 131-133) zugleich zur Verbindung mit der wenigstens einen mittels der Ausformung (42, 43, 49, 490) gegen den Nagelschaft (1) setzbaren Kopfkalotte (4) des Bausatzes ausgebildet ist.

2. BausatznachAnspruch 1, **dadurch gekennzeichnet, daß** an der Unterseite der genannten Kopfkalotte (4) wenigstens eine genannte, eine Führung bildende Ausformung (42, 43, 49, 490) ausgebildet ist, mittels der die Kalotte (4) im Aufsitz gegen den Nagelschaft (1) setzbar ist, und daß ein lateral an dem Nagelschaft (1) angreifendes Verbindungsmittel (51, 491) vorgesehen ist, das in gelöstem Zustand Kopfkalotte (4) und Nagelschaft (1) lose und unverlierbar mit Justier-Bewegungsspiel (V1, V2, D2) für die Kalotte (4) zum Ausrichten derselben miteinander verbindet, und das in gespanntem Zustand die Verbindung zwischen Kopfkalotte (4) und Nagelschaft (1) fixiert.

3. Bausatz nach Anspruch 2, **dadurch gekennzeichnet, daß** das lateral an dem Nagelschaft (1) angreifende, zum Ausrichten der Kopfkalotte (4) vorgesehene Verbindungsmittel ein einziges stabförmiges Verbindungselement (51, 53) zum losen und fixierenden Verbinden der Kopfkalotte (4) mit dem Nagelschaft (1) umfaßt.

4. Bausatz nach Anspruch 2 oder3, **dadurch gekennzeichnet, daß** der Bausatz wenigstens ein stabförmiges Verbindungselement (51, 52, 53) zum Verbinden der Kopfkalotte (4) mit dem Nagelschaft (1) umfaßt.

5. Bausatz nach Anspruch 3 oder 4, **dadurch gekennzeichnet, daß** an dem genannten Nagelschaft (1) wenigstens eine Verbindungs-Querdurchbohrung (14) derart angeordnet und aufgeweitet ausgebildet ist, daß sie ein zugehöriges genanntes stabförmiges Verbindungselement (51, 52) mit ausgeprägtem Justier-Bewegungsspiel (V1, V2, D2) von lateral her führt und aufnimmt.

6. Bausatz nach einem der Ansprüche 3 bis 5, **dadurch gekennzeich**n e t , daß eine mit einem genannten stabförmigen Verbindungselement (51, 52) an dem Nagelschaft (1) festsetzbare Kopfkalotte (4) mit einer die Ausformung an der Kalottenunterseite (400) bildenden Sitzausnehmung (42, 43) zur formschlüssigen Verbindung mit dem Marknagelschaft (1) versehen ist.

7. Bausatz nach einem der Ansprüche 3 bis 6, **dadurch gekennzeich**n e t , daß der Bausatz wenigstens eine genannte Osteosyntheseeinrichtung (6) mit zugehörigem Nagelschaft (1) umfaßt, in dem wenigstens eine Verbindungs-Querdurchbohrung (13, 13', 14, 133) für ein zugehöriges genanntes stabförmiges Verbindungselement (51, 52, 52') so angeordnet und ausgerichtet ist, daß die Verbindungs-Querdurchbohrung (13, 13', 14) wahlweise auch zur Aufnahme eines Versorgungs-Stabelements (23, 23', 24), vorzugsweise in Form einer Versorgungsschraube, nutzbar ist.

8. Bausatz nach einem der Ansprüche 3 bis 7, **dadurch gekennzeich**n e t , daß der Bausatz wenigstens eine genannte Osteosyntheseeinrichtung (6) mit Nagelschaft (1) umfaßt, in dem wenigstens eine Verbindungs-Querdurchbohrung (14) für das zugehörige genannte stabförmige Verbindungselement (51) so angeordnet und ausgerichtet ist, daß dieses Verbindungselement (51) im Bereich in der Humerus-Frontalmittelebene (AP-Ebene) (800) zu liegen kommt.

9. Bausatz nach einem der Ansprüche 3 bis 8, **dadurch gekennzeichnet, daß** der Bausatz wenigstens eine genannte Osteosyntheseeinrichtung (6) umfaßt, deren Nagelschaft (1) eine Verbindungs-Querdurchbohrung (14) für das stabförmige Verbindungselement (51) aufweist, die in dem mit dem Tuberculum majus (83) des Humerus (8) korrespondierenden proximalen Endabschnitt (16) des Nagelschafts (1) ausgebildet ist.

10. Bausatz nach einem der Ansprüche 3 bis 9, **dadurch gekennzeich**n e t , daß der Bausatz wenigsten eine Kopfkalotte (4) mit einer Unterseite (400) umfaßt, an der eine genannte Ausformung (42, 43, 49) ausgebildet ist, die mit einem korrespondierenden, dem Nagelschaft (1) zugeordneten Lagerteil zur Höhenverstellung der Kopfkalotte (4) bewegbar verbunden ist, wobei an der Kalotten-Unterseite (400) wenigstens eine Bohrung (41, 45, 45') zur kraftschlüssigen Aufnahme des medialen Endes des zugehörigen stabförmigen Verbindungselements (51, 52, 52') ausgebildet ist.

11. Bausatz nach einem der Ansprüche 1 bis 10, **dadurch gekennzeich**n e t , daß der Bausatz wenigstens ein Verbindungs-Adapterstück (9) umfaßt, das Nagelschaft (1) und Kopfkalotte (4) derart miteinander verbindet, daß die Kopfkalotte (4) wenigstens in einer Raumrichtung (V2, D1, D2) in ihrer Position relativ zum Nagelschaft (1) verstellbar und feststellbar ist.

12. Bausatz nach einem der Ansprüche 1 bis 11, **dadurch gekennzeichnet, daß** der Bausatz wenigstens eine Kopfkalotte (4) umfaßt, an deren Unterseite (400) als Bestandteil des Verbindungsmittels ein halsförmiges Ansatzstück (440) zur klemmenden Verbindung mit einem genannten Nagelschaft (1) des Bausatzes ausgebildet ist, wobei das Ansatzstück (440) ein eine Ausnehmung bildendes Steckloch (490) mit wenigstens einem lateralen Wandelement (491) aufweist, das den Nagelschaft (1) im Zustand der Steckverbindung hintergreift.

13. Bausatz nach einem der Ansprüche 1 bis 12, **dadurch gekennzeich**n e t , daß der Bausatz wenigstens eine genannte Osteosyntheseeinrichtung (6) mit zugehörigem Nagelschaft (1) umfaßt, der eine Gruppe von Versorgungs-Querdurchbohrungen (11, 12, 13, 13') aufweist, die derart angeordnet und ausgerichtet sind, daß zu der Osteosyntheseeinrichtung (6) zugehörige Versorgungsschrauben (21, 22; 23, 23') typische Muster kapitaler und subkapitaler Humerusfrakturen (86-88) erfassen.

14. Bausatz nach einem der Ansprüche 1 bis 13, **dadurch gekennzeich**n e t , daß der Bausatz wenigstens eine genannte Osteosyntheseeinrichtung (6) umfaßt, deren Nagelschaft (1) in einem Zwischenabschnitt (17), der mit dem chirurgischen Halsbereich (Collum chirurgicum) (82) des Humerus (8) und einem distalwärts daran angrenzenden Abschnitt korrespondiert, wenigstens eine Versorgungs-Querdurchbohrung (11, 12, 13, 13') für ein Versorgungs-Stabelement (21, 22, 23, 23') aufweist, die so angeordnet und ausgerichtet ist, daß das Versorgungs-Stabelement (21, 22, 23, 23') jeweils in dem medialen Bereich des Humeruskopfes (85) zu liegen kommt.

15. Bausatz nach einem der Ansprüche 1 bis 14, **dadurch gekennzeich**n e t , daß der Bausatz wenigstens eine genannte Osteosyntheseeinrichtung (6) umfaßt, deren Nagelschaft (1) in einem Zwischenabschnitt (17), der mit dem Halsbereich (Collum chirurgicum) (82) des Humerus (8) und einem distalwärts daran angrenzenden Abschnitt korrespondiert, wenigstens eine Versorgungs-Querdurchbohrung (11, 12, 13, 13') aufweist, die so angeordnet und ausgerichtet ist, daß wenigstens ein von der zugehörigen Versorgungs-Querdurchbohrung (11, 12, 13, 13') geführtes Versorgungs-Stabelement (21, 22, 23, 23') mit Ausrichtung nach dorsal und/oder ventral in dem Humeruskopf (85) zu liegen kommt.

16. Bausatz nach Anspruch 15, **dadurch gekennzeichnet, daß** in dem Schaft-Zwischenabschitt (17) wenigstens zwei Versorgungs-Querdurchbohrungen (11, 12; 13, 13') für Versorgungs-Stabelemente (21, 22; 23, 23') ausgebildet sind, die sich längs des Nagelschafts (1) nebeneinander und unter spitzem Winkel (26) kreuzend erstrecken.

17. Bausatz nach einem der Ansprüche 1 bis 16, **dadurch gekennzeich**n e t , daß der Bausatz wenigstens eine genannte Osteosyntheseeinrichtung (6) umfaßt, deren Nagelschaft (1) eine proximale Stirnfläche (15) aufweist und so geformt ist, daß sich die proximale Stirnfläche (15) des in den Humerus (8) eingebrachten Nagelschafts (1) an der oberen proximalen Seite des Humerus (8) in freiem operativem Zugriff befindet.

18. Bausatz nach Anspruch 17, **dadurch gekennzeichnet, daß** sich der Nagelschaft (1), ausgehend von seinem dem Hals (Collum chirurgicum) (82) entsprechenden Schaftabschnitt (18), zu seinem proximalen Ende hin entsprechend der lateralwärts gerichteten Krümmung des Humerus (8) gekrümmt erstreckt.

19. Bausatz nach einem der Ansprüche 1 bis 18, **dadurch gekennzeichnet, daß** die Kopfkalotte (4) außerhalb eines an ihrer Unterseite (400) ausgebildeten Anschlußstückes (44, 48, 440) weitgehend hohlförmig ausgebildet ist.

## Claims

1. Kit for the treatment of capital and subcapital humerus fractures (86-88) comprising the following individual implant parts belonging to one another:
- at least one nail shaft (1) extending with its longitudinal axis (10) and which can be introduced proximally into the bone cavity of the humerus (8) and which has at least one transverse treatment through-bore (11-14, 131-133) for at least one associated treatment screw (21-24) for treating capital and/or subcapital humerus fractures (86-88);
- at least one head cap (4) formed as a humerus prosthetic articular head which can be placed against the nail shaft (1) by means of moulding (42, 43, 49, 490) and can be attached to the proximal end of the nail shaft (1);
- at least one connecting means for connecting each head cap to the nail shaft (1) and
- at least one treatment screw (21-24) and at least one locking screw (31-33),
wherein the said nail shaft (1), which is a proportionate constituent of an intramedullary osteosynthesis device (6) which comprises the nail shaft (1), the at least one treatment screw (21-24) and at least one locking screw (31-33), is designed on its proximal end in the region of at least of one said transverse treatment through-bores (11-14, 131-133) at the same time for connection to the at least one head cap (4) of the kit which can be placed against the nail shaft (1) by means of the moulding (42, 43, 49, 490).

2. Kit according to claim 1, **characterised in that** on the underside of the said head cap (4), is designed at least one said moulding (42, 43, 49, 490) forming a guide and by means of which the cap (4) can be placed mounted against the nail shaft (1), and **in that** a connecting means (51, 491) contacting the nail shaft (1) laterally is provided which in the released state connects head cap (4) and nail shaft (1) loosely and undetachably with adjusting movement play (V1, V2, D2) for the cap (4) to align the same with one another and which in the tightened state, fixes the connection between head cap (4) and nail shaft (1).

3. Kit according to claim 2, **characterised in that** the connecting means provided for aligning the head cap (4) and contacting the nail shaft (1) laterally comprises a single rod-like connecting element (51, 53) for loose and fixing connection of the head cap (4) with the nail shaft (1).

4. Kit according to claim 2 or 3, **characterised in that** the kit comprises at least one rod-like connecting element (51, 52, 53) for connecting the head cap (4) to the nail shaft (1).

5. Kit according to claim 3 or 4, **characterised in that** on the said nail shaft (1) is formed at least one transverse treatment through-bore (14) arranged and expanded such that it guides and receives laterally an associated said rod-like connecting element (51, 52) with pronounced adjusting movement clearance (V1, V2, D2).

6. Kit according to any one of claims 3 to 5, **characterised in that** a head cap (4) which can be fixed to the nail shaft (1) using a said rod-like connecting element (51, 52) is provided with a seat recess (42, 43) forming the moulding on the cap underside (400) for positive locking with the medulla nail shaft (1).

7. Kit according to any one of claims 3 to 6, **characterised in that** the kit comprises at least one said osteosynthesis device (6) with associated nail shaft (1), in which at least one transverse connecting through-bore (13, 13', 14, 133) for an associated said rod-like connecting element (51, 52, 52') is arranged and aligned so that the transverse connecting through-bore (13, 13', 14) can be used alternatively also to receive a treatment rod element (23, 23', 24), preferably in the form of a treatment screw.

8. Kit according to any one of claims 3 to 7, **characterised in that** the kit comprises at least one said osteosynthesis device (6) with nail shaft (1), in which at least one transverse connecting throught-bore (14) for the associated said rod-like connecting element (51) is arranged and aligned so that this connecting element (51) comes to rest in the region in the humerus central frontal plane (AP plane) (800).

9. Kit according to any one of claims 3 to 8, **characterised in that** the kit comprises at least one said osteosynthesis device (6), the nail shaft (1) of which has a transverse connecting through-bore (14) for the rod-like connecting element (51), which is formed in the proximal end section (16) of the nail shaft (1) corresponding to the greater tuberosity (83) of the humerus (8).

10. Kit according to any one of claims 3 to 9, **characterised in that** the kit comprises at least one head cap (4) with an underside (400), on which a said moulding (42, 43, 49) is formed, which is connected to be movable to a corresponding bearing part assigned to the nail shaft (1) for height adjustment of the head cap (4), wherein at least one bore (41, 45, 45') for non-positive receiving of the medial end of the associated rod-like connecting element (51, 52, 52') is formed on the underside (400) of the cap.

11. Kit according to any one of claims 1 to 10, **characterised in that** the kit comprises at least one connecting adapter piece (9), which connects nail shaft (1) and head cap (4) to one another such that the head cap (4) at least in one spatial direction (V2, D1, D2) can be adjusted and fixed in its position relative to the nail shaft (1).

12. Kit according to any one of claims 1 to 11, **characterised in that** the kit comprises at least one head cap (4), on the underside (400) of which as a constituent of the connecting means, is formed a neck-like extension (440) for clamping connection with a said nail shaft (1) of the kit, wherein the extension (440) has a plug-in hole (490) forming a recess and with at least one lateral wall element (491), which engages behind the nail shaft (1) in the state of the plug-in connection.

13. Kit according to any one of claims 1 to 12, **characterised in that** the kit comprises at least one said osteosynthesis device (6) with associated nail shaft (1), which has a group of transverse treatment through-bores (11, 12, 13, 13') which are arranged and aligned such that treatment screws (21, 22; 23, 23') belonging to the osteosynthesis device (6) register typical patterns of capital and subcapital humerus fractures (86-88).

14. Kit according to any one of claims 1 to 13, **characterised in that** the kit comprises at least one said osteosynthesis device (6), the nail shaft (1) of which in an intermediate section (17), which corresponds to the surgical neck region (collum chirurgicum) (82) of the humerus (8) and a section adjoining distally thereto, has at least one transverse treatment through-bore (11, 12, 13, 13') for a treatment rod element (21, 22, 23, 23') which is arranged and aligned so that the treatment rod element (21, 22, 23, 23') comes to rest in each case in the medial region of the humerus head (85).

15. Kit according to any one of claims 1 to 14, **characterised in that** the kit comprises at least one said osteosynthesis device (6), the nail shaft (1) of which in an intermediate section (17), which corresponds to the neck region (collum chirurgicum) (82) of the humerus (8) and a section adjoining distally thereto, has at least one transverse treatment through-bore (11, 12, 13, 13') which is arranged and aligned so that at least one treatment rod element (21, 22, 23, 23') guided by the associated transverse treatment through-bore (11, 12, 13, 13') comes to rest in the humerus head (85) with alignment dorsally and/or ventrally.

16. Kit according to claim 15, **characterised in that** in the shaft intermediate section (17) are formed at least two transverse treatment through-bores (11, 12; 13, 13') for treatment rod elements (21, 22; 23, 23') which extend along the nail shaft (1) next to one another and crossing at an acute angle (26).

17. Kit according to any one of claims 1 to 16, **characterised in that** the kit comprises at least one said osteosynthesis device (6), the nail shaft (1) of which has a proximal end face (15) and is shaped so that the proximal end face (15) of the nail shaft (1) introduced into the humerus (8) is in free operative access on the upper proximal side of the humerus (8).

18. Kit according to claim 17, **characterised in that** the nail shaft (1), starting from its shaft section (18) corresponding to the neck (collum chirurgicum) (82), extends in curved manner towards its proximal end corresponding to the laterally directed curvature of the humerus (8).

19. Kit according to any one of claims 1 to 18, **characterised in that** the head cap (4) is formed to be largely hollow-like outside of a connecting piece (44, 48, 440) formed on its underside (400).

## Revendications

1. Trousse pour le traitement de fractures capitales et sous-capitales de l'humérus (86-88), comportant les éléments d'implant suivants faisant partie les uns des autres :
- au moins une tige de clou (1) pouvant être introduite de façon proximale dans la cavité médullaire de l'humérus (8), s'étendant longitudinalement avec son axe longitudinal (10) et comprenant au moins un trou traversant transversal de soutien (11-14, 131-133) pour au moins une vis de soutien associée (21-24) servant à soutenir des fractures capitales et/ou sous-capitales de l'humérus (86-88) ;
- au moins une coiffe (4) pouvant être fixée sur l'extrémité proximale de la tige de clou (1), posée contre la tige de clou (1) au moyen d'une protubérance (42, 43, 49, 490), et conçue comme une tête d'articulation prothétique d'humérus ;
- au moins un moyen de liaison servant à relier chaque coiffe à la tige de clou (1) et
- au moins une vis de soutien (21-24) et au moins une vis de verrouillage (31-33),
ladite tige de clou (1) faisant partie intégrante d'un dispositif d'ostéosynthèse intramédullaire (6), qui comprend la tige de clou (1) comportant au moins une vis de soutien (21-24) et au moins une vis de verrouillage (31-33), étant conçue sur son extrémité proximale dans la zone d'au moins un dit trou traversant transversal de soutien (11-14, 131-133) pour la liaison simultanée à la ou aux coiffes (4) de la trousse pouvant être posées contre la tige de clou (1) au moyen de la protubérance (42, 43, 49, 490).

2. Trousse selon la revendication 1, **caractérisée en ce qu'**au moins une dite protubérance (42, 43, 49, 490) au moyen de laquelle la coiffe (4) peut être posée bien droite contre la tige de clou (1) et formant un guidage est conçue sur le côté inférieur de ladite coiffe (4), et **en ce qu'**il est prévu un moyen de liaison (51, 491) latéralement appliqué sur la tige de clou (1), reliant dans l'état desserré la coiffe (4) et la tige de clou (1) l'une à l'autre de façon desserrée et solide avec un jeu de déplacement d'alignement (V1, V2, D2) pour la coiffe (4) afin d'orienter ladite coiffe, et fixant dans l'état serré la liaison entre la coiffe (4) et la tige de clou (1).

3. Trousse selon la revendication 2, **caractérisée en ce que** le moyen de liaison s'appliquant latéralement sur la tige de clou (1) et conçu pour orienter la coiffe (4) comporte un seul élément de liaison en forme de barre (51, 53) servant à relier de manière desserrée et solide la coiffe (4) à la tige de clou (1).

4. Trousse selon la revendication 2 ou 3, **caractérisée en ce que** la trousse comporte au moins un élément de liaison en forme de barre (51, 52, 53) servant à relier la coiffe (4) à la tige de clou (1).

5. Trousse selon la revendication 3 ou 4, **caractérisée en ce qu'**au moins un trou traversant transversal de liaison (14) est disposé et élargi sur ladite tige de clou (1) de telle sorte qu'il guide depuis le côté latéral et loge un dit élément de liaison en forme de barre (51, 52) associé avec un jeu de déplacement d'alignement (V1, V2, D2) prononcé.

6. Trousse selon l'une quelconque des revendications 3 à 5, **caractérisée en ce qu'**une coiffe (4) pouvant être fixée sur la tige de clou (1) à l'aide d'un dit élément de liaison en forme de barre (51, 52) est pourvue d'un évidement de siège (42, 43) formant la protubérance sur le côté inférieur de calotte (400) et servant à la liaison par complémentarité de forme à la tige de clou médullaire (1).

7. Trousse selon l'une quelconque des revendications 3 à 6, **caractérisée en ce que** la trousse comporte au moins un dit dispositif d'ostéosynthèse (6) doté d'une tige de clou (1) associée dans laquelle au moins un trou traversant transversal de liaison (13, 13', 14, 133) pour un dit élément de liaison en forme de barre (51, 52, 52') associé est disposé et orienté de sorte que le trou traversant transversal de liaison (13, 13', 14) peut être également utilisé au choix pour loger un élément de soutien en forme de barre (23, 23', 24), de préférence sous forme d'une vis de soutien.

8. Trousse selon l'une quelconque des revendications 3 à 7, **caractérisée en ce que** la trousse comporte au moins un dit dispositif d'ostéosynthèse (6) doté d'une tige de clou (1) dans laquelle au moins un trou traversant transversal de liaison (14) pour ledit élément de liaison en forme de barre (51) associé est disposé et orienté de sorte que cet élément de liaison (51) vient se placer dans la zone du plan médian frontal de l'humérus (plan AP) (800).

9. Trousse selon l'une quelconque des revendications 3 à 8, **caractérisée en ce que** la trousse comporte au moins un dit dispositif d'ostéosynthèse (6) dont la tige de clou (1) comprend un trou traversant transversal de liaison (14) pour l'élément de liaison en forme de barre (51), ledit trou étant conçu dans la partie d'extrémité proximale (16) de la tige de clou (1) correspondant au tubercule majeur (83) de l'humérus (8).

10. Trousse selon l'une quelconque des revendications 3 à 9, **caractérisée en ce que** la trousse comporte au moins une coiffe (4) dotée d'un côté inférieur (400) sur lequel est conçue une dite protubérance (42, 43, 49) reliée mobile à une partie de support correspondante associée à la tige de clou (1) pour le réglage en hauteur de la coiffe (4), au moins un trou (41, 45, 45') servant au logement à force de l'extrémité médiale de l'élément de liaison en forme de barre (51, 52, 52') associé étant conçu sur le côté inférieur de calotte (400).

11. Trousse selon l'une quelconque des revendications 1 à 10, **caractérisée en ce que** la trousse comporte au moins une pièce adaptatrice de liaison (9) reliant la tige de clou (1) et la coiffe (4) l'une à l'autre de telle sorte que la coiffe (4) peut être déplacée au moins dans une direction spatiale (V2, D1, D2) et immobilisée dans sa position par rapport à la tige de clou (1).

12. Trousse selon l'une quelconque des revendications 1 à 11, **caractérisée en ce que** la trousse comporte au moins une coiffe (4) sur le côté inférieur (400) de laquelle une embouchure en forme de col (440) faisant partie du moyen de liaison est conçue pour la liaison par serrage à une dite tige de clou (1) de la trousse, l'embouchure (440) comprenant un trou d'insertion (490) formant un évidement et doté d'au moins un élément latéral de paroi (491) venant se bloquer derrière la tige de clou (1) dans l'état de la liaison par insertion.

13. Trousse selon l'une quelconque des revendications 1 à 12, **caractérisée en ce que** la trousse comporte au moins un dit dispositif d'ostéosynthèse (6) doté d'une tige de clou (1) associée comprenant un groupe de trous traversants transversaux de soutien (11, 12, 13, 13') disposés et orientés de telle sorte que les vis de soutien (21, 22 ; 23, 23') faisant partie du dispositif d'ostéosynthèse (6) détectent des formes habituelles des fractures capitales et sous-capitales de l'humérus (86-88).

14. Trousse selon l'une quelconque des revendications 1 à 13, **caractérisée en ce que** la trousse comporte au moins un dit dispositif d'ostéosynthèse (6) dont la tige de clou (1) comprend dans une partie intermédiaire (17) correspondant à la zone du cou chirurgical (82) de l'humérus (8) et dans une partie adjacente à celle-ci de manière distale au moins un trou traversant transversal de soutien (11, 12, 13, 13') pour un élément de soutien en forme de barre (21, 22, 23, 23'), ledit trou étant disposé et orienté de sorte que l'élément de soutien en forme de barre (21, 22, 23, 23') vient se placer respectivement dans la zone médiale de la tête de l'humérus (85).

15. Trousse selon l'une quelconque des revendications 1 à 14, **caractérisée en ce que** la trousse comporte au moins un dit dispositif d'ostéosynthèse (6) dont la tige de clou (1) comprend dans une partie intermédiaire (17) correspondant à la zone de cou (cou chirurgical) (82) de l'humérus (8) et une partie adjacente à celle-ci de manière distale au moins un trou traversant transversal de soutien (11, 12, 13, 13') disposé et orienté de sorte qu'au moins un élément de soutien en forme de barre (21, 22, 23, 23') guidé par le trou traversant transversal de soutien (11, 12, 13, 13') associé vient se placer dans la tête de l'humérus (85) avec une orientation dorsale et/ou ventrale.

16. Trousse selon la revendication 15, **caractérisée en ce qu'**au moins deux trous traversants transversaux de soutien (11, 12 ; 13, 13') pour des éléments de soutien en forme de barre (21, 22 ; 23, 23') juxtaposés le long de la tige de clou (1) et se croisant en formant un angle aigu (26) sont conçus dans la partie intermédiaire de tige (17).

17. Trousse selon l'une quelconque des revendications 1 à 16, **caractérisée en ce que** la trousse comporte au moins un dit dispositif d'ostéosynthèse (6) dont la tige de clou (1) comprend une surface frontale proximale (15) et est formée de sorte que la surface frontale proximale (15) de la tige de clou (1) introduite dans l'humérus (8) se situe sur le côté proximal supérieur de l'humérus (8) en accès opératoire libre.

18. Trousse selon la revendication 17, **caractérisée en ce que** la tige de clou (1), à partir de sa partie de tige (18) correspondant au cou (cou chirurgical) (82), s'étend en direction de son extrémité proximale suivant la courbure orientée latéralement de l'humérus (8).

19. Trousse selon l'une quelconque des revendications 1 à 18, **caractérisée en ce que** la coiffe (4) est en grande partie creuse et conçue à l'extérieur d'une embouchure (44, 48, 440) conçue sur son côté inférieur (400).
